# EUROPEAN PATENT APPLICATION

(11) **EP 4 678 760 A1**
(43) Date of publication of application: **14.01.2026**
(21) Application number: 24789080.9
(22) Date of filing: 12.04.2024
(51) Int. Cl.: C12P 13/06, C12N 15/77, C12N 9/02

(54) **MICROORGANISM HAVING INCREASED ACTIVITY OF PYRUVATE DEHYDROGENASE COMPLEX SUBUNIT E1, AND METHOD FOR PRODUCING O-ACETYLHOMOSERINE OR DERIVATIVE THEREOF BY USING SAME**

(30) Priority: 12.04.2023 KR 20230048233
(71) Applicant: CJ Cheiljedang Corporation, Seoul 04560 (KR)
(72) Inventor: KIM, Ju Eun, Seoul 04560 (KR); KIM, Ye-Eun, Seoul 04560 (KR)
(74) Representative: Meissner Bolte Partnerschaft mbB
(86) International application number: PCT/KR2024/004952
(87) International publication number: WO 2024/215139

(57) **Abstract**

The present disclosure relates to a microorganism having an increased activity of pyruvate dehydrogenase complex subunit E1, and a method for producing O-acetylhomoserine or a derivative thereof using the same.

## Description

### [Technical Field]

The present disclosure relates to a microorganism having an increased activity of pyruvate dehydrogenase complex subunit E1, and a method for producing O-acetylhomoserine or a derivative thereof using the same.

### [Background Art]

A microorganism of the genus *Corynebacterium* is a Gram-positive microorganism that is widely used in the production of L-amino acids, and is widely used to produce L-homoserine, L-methionine, *etc.,* which can be converted from O-acetylhomoserine, in addition to O-acetylhomoserine, which is classified as a non-proteinaceous amino acid as well as a proteinaceous amino acid.

For the production of L-amino acid and other useful materials, various studies are underway to develop microorganisms with high-efficiency production. For example, target material specific approaches (*e.g*., increasing the expression of genes encoding the enzymes involved in the biosynthesis of L-amino acids or removing genes unnecessary for biosynthesis of L-amino acids mainly in the strains of the genus *Corynebacterium*) are mainly used for the production of L-amino acids (US 9644009 B2).

### [Disclosure]

### [Technical Problem]

There is still a demand for research on methods to effectively produce L-amino acids with a high yield.

### [Technical Solution]

It is one object of the present disclosure to provide a microorganism having an increased activity of pyruvate dehydrogenase complex subunit E1 (AceE).

In one embodiment, the microorganism is a microorganism for producing O-acetylhomoserine or a derivative thereof.

In another embodiment, the microorganism has activity of pyruvate dehydrogenase complex subunit E1 (AceE), which is increased compared to the endogenous activity thereof.

As the microorganism according to any one of the above-described embodiments, the microorganism may be a microorganism of the genus *Corynebacterium.*

As the microorganism according to any one of the above-described embodiments, the microorganism of the genus *Corynebacterium* may be *Corynebacterium glutamicum.*

As the microorganism according to any one of the above-described embodiments, the pyruvate dehydrogenase complex subunit E1 may be derived from *Corynebacterium glutamicum.*

As the microorganism according to any one of the above-described embodiments, the pyruvate dehydrogenase complex subunit E1 may include an amino acid sequence of SEQ ID NO: 1.

As the microorganism according to any one of the above-described embodiments, the pyruvate dehydrogenase complex subunit E1 may be encoded by a polynucleotide of SEQ ID NO: 2 or SEQ ID NO: 3.

As the microorganism according to any one of the above-described embodiments, the microorganism may be one having an increased ability to produce O-acetylhomoserine or a derivative thereof compared to a non-modified microorganism.

As the microorganism according to any one of the above-described embodiments, O-acetylhomoserine or a derivative thereof may be any one or more selected from O-acetylhomoserine, L-methionine, and L-homoserine.

It is another object of the present disclosure to provide a method for producing O-acetylhomoserine or a derivative thereof, including: culturing a microorganism having an increased activity of pyruvate dehydrogenase complex subunit E1 is in a medium.

In one embodiment, the method may further include a step of recovering O-acetylhomoserine or a derivative thereof from the cultured microorganism, a culture of the microorganism, a fermented product of the microorganism, or the culture medium.

It is still another object of the present disclosure to provide a composition for producing O-acetylhomoserine or a derivative thereof, including: a microorganism, in which the activity of pyruvate dehydrogenase complex subunit E1 is increased compared to the endogenous activity thereof; a culture of the microorganism; a fermented product of the microorganism; or a combination of two or more thereof.

It is still another object of the present disclosure to provide use of a microorganism, in which the activity of pyruvate dehydrogenase complex subunit E1 is increased compared to the endogenous activity thereof, for the production of O-acetylhomoserine or a derivative thereof.

It is still another object of the present disclosure to provide a method for preparing a microorganism for producing O-acetylhomoserine or a derivative thereof, including: modifying the microorganism to have an increased activity of pyruvate dehydrogenase complex subunit E1 compared to the endogenous activity thereof.

### [Advantageous Effects]

O-acetylhomoserine or a derivative thereof can be produced with a high yield using the microorganism of the present disclosure.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Additionally, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

### Definitions

As used in the specification and appended claims, the singular forms ("a", "an", and "the") include plural referents unless the context clearly states otherwise. Unless the context indicates otherwise, singular terms shall include the plural and plural terms shall include the singular. As used in the specification and appended claims, unless stated otherwise, the use of "or" may be used to include "and/or.

As used herein, the term "about" may be presented before a particular numerical value. The term "about" used herein includes not only the exact number recited after the term, but also a range that is near or close to that number. Considering the context in which the number is presented, it can be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third..." "i), ii), iii)..." or "(a), (b), (c), (d)..." may be used to distinguish respective constitutions. When the terms are used in reference to steps of a method, use, or assay, there is no limitation that these terms are performed continually or sequentially. For example, there may be no time interval between these steps, or they may be performed concurrently, or may be performed with several seconds, several minutes, several hours, several days, or several months apart.

As used herein, the term "consisting essentially of" may mean that when the features of the object claimed herein are not substantially affected by the presence of unspecified features, the unspecified features may be present.

As used herein, the term, "consisting of" means that the total ratio of specific component(s) recited after the term is 100%. The components or features that are recited after the term "consisting of" may be essential or mandatory. In some embodiments, in addition to the components or features recited after the term "consisting of", any other components or features, or non-essential components or features may be excluded.

As used herein, the term "comprising/including" means the presence of features, steps, or constituting elements recited after the term, and does not exclude the presence of additional one or more features, steps, or constituting elements. The components or features recited after the term "comprising/including" herein may be essential or mandatory. However, in some embodiments, the term may further include any other or non-essential components or features.

### Proteins, Polypeptides

As used herein, the term "protein" or "polypeptide" refers to a polymer or oligomer of consecutive amino acid residues. In the present disclosure, the "polypeptide", "protein", and "peptide" may be used interchangeably with each other.

In some cases, a protein, polypeptide or peptide exhibiting activity may be referred to as an "enzyme". In the present disclosure, unless indicated otherwise, the amino acid sequences are described in an N-terminal to C-terminal direction.

As used herein, the term "mature polypeptide" means a polypeptide in a form without a signal sequence or pro-peptide sequence. A mature protein/polypeptide/peptide may be a functional form of a protein/polypeptide/peptide. The mature polypeptide may refer to a polypeptide in a final form after translation, or post-translational modification. Examples of the post-translational modification include N- or C-terminal modification, glycosylation, phosphorylation, leader sequence removal *etc.,* but are not limited thereto.

With respect to the amino acid sequences in the present disclosure, although it is described as a polypeptide or protein "comprising/including" an amino acid sequence described by a specific sequence number, a polypeptide or protein "consisting of" an amino acid sequence described by a specific sequence number, or a polypeptide or protein "having" an amino acid sequence described by a specific sequence number, it is apparent that any polypeptide or protein having an amino acid sequence in which part of the sequence(s) is deleted, modified, substituted, conservatively substituted or added may fall within the scope of the present disclosure if it has activity identical or corresponding to the polypeptide or protein consisting of the amino acid sequence of the corresponding sequence number. For example, polypeptides or proteins having sequence addition or deletion that do not alter the function of the protein, naturally occurring mutations, silent mutations thereof, or conservative substitutions within, or upstream or downstream (N-terminal or C-terminal) of the polypeptide or protein sequences may be included, as long as they have activity identical or corresponding to the activity of the polypeptide or protein.

In one specific example, polypeptides (proteins) that may be conjugated with a signal (or leader) sequence at the N-terminal involved in the translocation of polypeptides (proteins) co-translationally or post-translationally, or polypeptides (proteins) that may be conjugated with another sequence or linker to identify, purify, or synthesize the polypeptides (proteins) may also fall within the scope of the polypeptide of the amino acid sequence described by the specific number.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. The amino acids may be classified into the following groups: In one example, positively charged (basic) amino acids include arginine, lysine, and histidine; negatively charged (acidic) amino acids include glutamic acid and aspartic acid; amino acids having nonpolar side chains (nonpolar amino acids) include glycine, alanine, valine, leucine, isoleucine, methionine, phenylalanine, tryptophan, and proline; amino acids having polar or hydrophilic side chains (polar amino acids) include serine, threonine, cysteine, tyrosine, asparagine, and glutamine. In another example, amino acids having electrically charged side chains (electrically charged amino acids) include arginine, lysine, histidine, glutamic acid, and aspartic acid; and amino acids having uncharged side chains (uncharged amino acid; also referred to as neutral amino acids) include glycine, alanine, valine, leucine, isoleucine, methionine, phenylalanine, tryptophan, proline, serine, threonine, cysteine, tyrosine, asparagine, and glutamine. In still another example, aromatic amino acids include phenylalanine, tryptophan, and tyrosine. In yet another example, branched amino acids include valine, leucine, and isoleucine. In even another example, the 20 amino acids may be classified according to their size into 5 groups, starting from the amino acid groups with a relatively small volume, i.e., glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, and tyrosine, but the classification of the amino acids is not limited thereto. Typically, conservative substitutions may have little or no effect on the activity of the polypeptide.

### Polynucleotides

As used herein, the term "gene" means a polynucleotide that encodes a polypeptide and a polynucleotide including regions the upstream and downstream of the coding region. In some embodiments, a gene may have a sequence (intron) inserted between each coding region (exon).

As used herein, the term "polynucleotide", nucleic acid, or nucleic acid molecule" which is a polymer of nucleotides composed of nucleotide monomers connected in a lengthy chain by a covalently bond, is a DNA (*e.g.*, cDNA or genomic DNA) or RNA (*e.g.*, mRNA) strand having at least a certain length.

### Homology, Identity

As used herein, the term "homology" or "identity" refers to the degree of relatedness between two given amino acid sequences or nucleotide sequences, and may be expressed as a percentage. The terms "homology" and "identity" may often be used interchangeably with each other.

The sequence homology or identity of conserved polynucleotides or polypeptides may be determined by standard alignment algorithms and may be used together with a default gap penalty established by the program being used. Substantially, homologous or identical sequences are generally expected to hybridize to all or at least about 50%, 50%, 60%, 70%, 80% or 90% of the entire length of the sequences under moderate or highly stringent conditions. Polynucleotides that contain degenerate codons instead of codons in the hybridizing polynucleotides may also be considered.

Whether any two polynucleotide or polypeptide sequences have a homology, similarity, or identity may be, for example, determined by a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277) (preferably, version 5.0.0 or versions thereafter) (GCG program package (Devereux, J., et al., Nucleic Acids Research 12: 387 (1984)), BLASTP, BLASTN, FASTA (Atschul, [S.] [F.,] [ET AL., J MOLEC BIOL 215]: 403 (1990); Guide to Huge Computers, Martin J. Bishop, [ED.,] Academic Press, San Diego, 1994, and [CARILLO ETA/.](1988) SIAM J Applied Math 48: 1073). For example, the homology, similarity, or identity may be determined using BLAST or ClustalW of the National Center for Biotechnology Information (NCBI).

The homology, similarity, or identity of polynucleotides or polypeptides may be, for example, determined by comparing sequence information using, for example, the GAP computer program, such as Needleman et al. (1970), J Mol Biol. 48: 443 as disclosed in Smith and Waterman, Adv. Appl. Math (1981) 2:482. In summary, the GAP program defines the homology, similarity, or identity as the value obtained by dividing the number of similarly aligned symbols (*i.e*., nucleotides or amino acids) by the total number of the symbols in the shorter of the two sequences. Default parameters for the GAP program may include (1) unitary matrices (containing a value of 1 for identities and 0 for non-identities), PAM Matrix as disclosed in Schwartz and Dayhoff, eds., Atlas Of Protein Sequence And Structure, National Biomedical Research Foundation (1978), and the weighted comparison matrix of Gribskov et al. (1986), Nucl. Acids Res. 14:6745 (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide sequences have homology, similarity, or identity may be determined by comparing sequences via Southern hybridization experiments under defined stringent conditions, and the appropriate hybridization conditions to be defined may be determined by way of a method within the scope of the present disclosure, which is known to those skilled in the art (*e.g*., J. Sambrook et al., Molecular Cloning, A Laboratory Manual; F.M. Ausubel et al., Current Protocols in Molecular Biology, John Wiley & Sons, Inc., New York), but is not limited thereto.

The "stringent conditions" refers to conditions under which specific hybridization between polynucleotides is allowed. Such conditions are specifically described in the literature (Sambrook et al., supra, 9.50-9.51, 11.7-11.8). For example, the stringent conditions may include conditions under which polynucleotides having high homology or identity, i.e., polynucleotides having 60% or more, 70% or more, 75% or more, 80% or more, 85% or more, 90% or more, 95% or more, 96% or more, 97% or more, 98% or more, or 99% or more homology or identity hybridize with each other, while polynucleotides having homology or identity lower than the above homology or identity do not hybridize with each other; or may include ordinary washing conditions of Southern hybridization, i.e., washing once, specifically twice or three times, at a salt concentration and a temperature corresponding to 60°C., 1×SSC, 0.1% SDS, specifically 60°C., 0.1×SSC, 0.1% SDS, and more specifically 68°C., 0.1×SSC, 0.1% SDS.

Hybridization requires that two nucleic acids have complementary sequences, although mismatches between bases are possible depending on the stringency of the hybridization. The term "complementary" is used to describe a relationship between nucleotide bases that may hybridize with each other. For example, with respect to DNA, adenine is complementary to thymine, and cytosine is complementary to guanine. Therefore, the polynucleotide of the present disclosure may also include an isolated nucleic acid fragment complementary to the entire sequence as well as a nucleic sequence substantially similar thereto.

For example, polynucleotides having a homology or identity to the polynucleotide of the present disclosure may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (*e.g*., Sambrook et al.).

### Nucleic Acid Construct, Vector, Transformation

As used herein, the term "nucleic acid construct" refers to a single- or doublestranded nucleic acid molecule, which includes one or more regulatory sequences, and which is artificially synthesized, or engineered to include a specific sequence in a manner that does not exist in nature, or isolated from nature.

As used herein, the term "Vector" refers to a DNA construct for delivering a target polynucleotide into a suitable host or host cell. In one example, the vector may contain the nucleotide sequence of a polynucleotide encoding the target polypeptide operably linked to a suitable expression regulatory region (expression regulatory sequence) so as to be able to express the target polypeptide in a suitable host cell, but is not limited thereto. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pDC, pBR, pUC, pBluescriptll, pGEM, pTZ, pCL and pET, *etc.* may be used. Specifically, pDZ, pDC, pACYC177, pACYC184, pCL, pECCG117, pUC19, pBR322, pMW118, pCC1BAC, pDCM2(WO2021-187781 A1) vector, *etc.* may be used.

In one example, a target polynucleotide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a target polynucleotide into a host cell to alter the genetic characteristics of the host cell. The transformed polynucleotide may be integrated into the chromosome of the host cell and located therein, or located extrachromosomally. Additionally, the polynucleotide may include DNA and/or RNA. The polynucleotide may be introduced in a suitable form depending on the purpose of introduction. For example, the polynucleotide for expressing the target polypeptide may be introduced into the host cell in the form of an expression cassette, which is a gene construct including all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into a host cell as it is and operably linked to sequences required for expression in the host cell, but is not limited thereto.

As used herein, the term "operably linked" refers to a constitution of placing a regulatory sequence to an appropriate position to regulate the expression of a coding sequence. Thus, the term "operably linked" includes an attachment or linking between a regulatory region of a functional domain having a known or desired activity such as a promoter, a stop codon, a signal sequence, or an enhancer, and a target (gene or polypeptide) such that the expression, secretion, or function of the target may be regulated in accordance with the known or desired activity. For example, it may mean that the polynucleotide sequence is functionally linked to a promoter sequence that initiates and mediates transcription of the polynucleotide encoding the polypeptide.

As used herein, the term "expression" includes any step involved in the production of a polypeptide, *e.g.*, transcription, post-transcriptional modification, translation, post-translational modification, and secretion, *etc.,* but is not limited thereto.

As used herein, the term "expression vector" refers to a linear or circular nucleic acid molecule including a target polynucleotide sequence and an operably linked regulatory sequence for expression thereof.

As used herein, the term "regulatory sequence" refers to a polynucleotide sequence necessary for the expression of a target polynucleotide sequence. Each regulatory sequence may be native (derived from the same origin) or foreign (derived from different genes) to the coding sequence, or may be a mutant sequence thereof or other artificial sequences. Examples of the regulatory sequence may include a leader sequence, a polyadenylation sequence, a pro-peptide sequence, a promoter, a signal peptide sequence, an operator sequence, a sequence encoding a ribosome-binding site, and a sequence for regulating the termination of transcription and translation. The minimum units of the regulatory sequence may include a promoter, and a sequence for terminating transcription and translation

As used herein, the term "recombinant" with respect to a cell, polynucleotide, polypeptide, or vector, means that the cell, polynucleotide, polypeptide or vector has been modified by the introduction of a heterologous nucleic acid or polypeptide or the alteration of a native polynucleotide or polypeptide, or that the cell is derived from a cell thus modified. Therefore, for example, recombinant cells may express genes that are not found within the native (non-recombinant) form of the cells, or may express native genes that are otherwise abnormally expressed, under expressed or not expressed at all.

### Microorganisms

As used herein, the term "microorganism (or strain)" includes all wild-type microorganisms, or prokaryotic or eukaryotic microorganisms in which natural or artificial genetic modifications occur, and it may be a microorganism in which a particular mechanism is weakened or enhanced due to insertion of a foreign gene, or enhancement or inactivation of the activity of an endogenous gene, *etc.,* and may be a microorganism including genetic modification to produce a desired polypeptide, protein or product. In the present disclosure, the "microorganism" and "strain" may be used interchangeably with each other.

For example, the microorganism of the present disclosure may be a microorganism (*e.g*., recombinant strain), in which the activity of pyruvate dehydrogenase complex subunit E1 is increased compared to the endogenous activity thereof, but is not limited thereto.

As used herein, the term "microorganism having the ability to produce O-acetylhomoserine or a derivative thereof", which is a microorganism capable of producing O-acetylhomoserine or a derivative thereof in an organism, may include all of microorganisms imparted with the ability to produce O-acetylhomoserine or a derivative thereof, which did not endogenously have the ability to produce O-acetylhomoserine or a derivative thereof, or microorganisms that endogenously have the ability to produce O-acetylhomoserine or a derivative thereof. The ability to produce O-acetylhomoserine or a derivative thereof may be imparted or enhanced by improvement of species.

As used herein, the term "non-modified microorganism (strain)" does not exclude a microorganism (strain) containing a mutation that may occur naturally, and may refer to a wild-type microorganism (strain) or natural-type microorganism (strain) itself, or a microorganism (strain) before its trait is altered due to genetic modification caused by natural or artificial factors. As used herein, the "non-modified microorganism (strain)" may be used interchangeably with "microorganism (strain) before modification", "non-mutant microorganism (strain)", "parent microorganism", "parent strain", "wild-type microorganism (strain)", "reference microorganism (strain)", or "standard microorganism (strain)". In the present disclosure, the non-modified microorganism may be a microorganism in which the activity of pyruvate dehydrogenase complex subunit E1 is not increased compared to the endogenous activity thereof, or a microorganism before the increase thereof.

Additionally, in the present disclosure, the non-modified microorganism may be a microorganism including a polynucleotide consisting of an amino acid sequence of SEQ ID NO: 1 or SEQ ID NO: 2 or SEQ ID NO: 3, but is not limited thereto.

### Increase of Polypeptide Activity

As used herein, the term "increase" of polypeptide activity means that the activity of a polypeptide is increased in a host cell, as compared to the endogenous activity thereof. The increase may be used interchangeably with terms such as activation, up-regulation, overexpression, enhancement, *etc.* The host cell may be a prokaryotic or eukaryotic microorganism.

The increase of polypeptide activity may include both cases in which a polypeptide activity not endogenously possessed by a host cell is exhibited, or a polypeptide activity is enhanced compared to the endogenous activity or the activity before modification.

For example, the description "exhibiting a polypeptide activity not endogenously possessed" may be caused by the "introduction of a polypeptide", but is not limited thereto. As used herein, the term "introduction" of polypeptide means that a gene not originally possessed by a microorganism is expressed in the microorganism, and thus the microorganism exhibits the activity of a particular polypeptide, or the activity of a polypeptide is increased or enhanced compared to the endogenous activity of the corresponding polypeptide or the activity before modification. For example, it may be a case in which a polynucleotide encoding a particular polypeptide is introduced into the chromosome of a microorganism, or a vector containing a polynucleotide encoding a particular polypeptide is introduced into a microorganism, thereby exhibiting its activity.

The "endogenous activity" refers to the activity of a particular polypeptide originally possessed by a microorganism before transformation or a non-modified microorganism when a trait is altered by genetic variation due to a natural or artificial factor. The endogenous activity may also be interchangeably used with "activity before modification".

The increase in the activity of a polypeptide as compared to the endogenous activity means that the activity and/or concentration (expression level) of the polypeptide of a microorganism is improved, as compared with the activity and/or concentration (expression level) of the polypeptide originally possessed by a microorganism before transformation or a non-modified microorganism.

In one example, the increase may mean that the activity of a corresponding protein/polypeptide not originally exhibited is exhibited, or the activity or concentration thereof is increased generally by about 1 % or more, about 10 % or more, about 25 % or more, about 50 % or more, about 75 % or more, about 100 % or more, about 150 % or more, about 200 % or more, about 300 % or more, about 400 % or more, or about 500 % or more, maximum about 1000 % or about 2000 % or more, based on the activity or concentration of the initial microbial strain, but is not limited thereto.

The increase of the activity of the polypeptide may be achieved by introducing a foreign polypeptide or increasing the activity of an endogenous polypeptide. Whether or not the activity of the polypeptide is increased may be confirmed from the activity level of the corresponding polypeptide, expression level thereof, or the increase in the amount of products produced from the corresponding polypeptide.

The increase of the activity of the polypeptide may be applied by various methods well known in the art, and is not limited as long as it can increase the activity of a target polypeptide compared to that of a microorganism before modification. Specifically, genetic engineering and/or protein engineering well known to those skilled in the art, which is a common method of molecular biology, may be used, but the method is not limited thereto (*e.g*., Sitnicka et al. Functional Analysis of Genes. Advances in Cell Biology. 2010, Vol. 2. 1-16, Sambrook et al. Molecular Cloning 2012, *etc.*)*.*

Specifically, the increase of the polypeptide activity of the present disclosure may be achieved by:
1) increasing the intracellular copy number of a polynucleotide encoding the polypeptide;
2) modifying the expression regulatory region of a gene encoding the polypeptide on the chromosome (*e.g*., inducing a modification within the expression regulatory region, replacing with a sequence having a stronger activity, or inserting a sequence having a stronger activity);
3) modifying the nucleotide sequence encoding the initiation codon or 5'-UTR of a gene transcript encoding the polypeptide;
4) modifying the amino acid sequence of the polypeptide such that the activity of the polypeptide is increased;
5) modifying the polynucleotide sequence encoding the polypeptide such that the activity of the polypeptide is increased (*e.g*., modifying the polynucleotide sequence of the polypeptide gene to encode a polypeptide that has been modified to increase the activity of the polypeptide);
6) introducing a foreign polypeptide exhibiting the polypeptide activity or a foreign polynucleotide encoding the same;
7) codon-optimization of a polynucleotide encoding the polypeptide;
8) analyzing the tertiary structure of the polypeptide and thereby selecting and modifying the exposed site, or chemically modifying the same;
9) regulating the cellular localization of the protein (polypeptide); or
10) a combination of two or more selected from above 1) to 9), but is not particularly limited thereto.

For example,
The 1) method of increasing the intracellular copy number of a polynucleotide encoding the polypeptide may be achieved by introducing a vector containing a polynucleotide encoding the polypeptide, which is operably linked to a suitable regulatory sequence, into a host cell. The vector may be a vector which is able to replicate and function regardless of a host cell. Alternatively, the method may be achieved by introducing one copy or two or more copies of polynucleotides encoding the polypeptide, which is operably linked to a suitable regulatory sequence, into the chromosome of the host cell. The introduction into the chromosome may be performed by introducing a vector, which is able to insert the polynucleotide into the chromosome of the host cell, into the host cell, but is not limited thereto. The vector is as described above. The regulatory sequence may be native (derived from the same origin) or foreign (derived from different genes) to the coding polynucleotide sequences, or may be a mutant sequence thereof or other artificial sequences, and may induce the expression of the polynucleotide in the host cell.

The 2) method of replacing the expression regulatory region (or expression regulatory sequence) of a gene encoding the polypeptide on the chromosome with a sequence having a strong activity may be achieved, for example, by inducing a modification on the sequence through deletion, insertion, substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome-binding site, and a sequence regulating the termination of transcription and translation, *etc.* In one example, the method may include replacing the original promoter with a strong promoter, but is not limited thereto

Examples of the known strong promoter may include cj1 to cj7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) method of modifying the nucleotide sequence encoding the initiation codon or 5'-UTR of a gene encoding the polypeptide may be achieved, for example, by substituting the initiation codon with another initiation codon having a higher expression rate of the polypeptide compared to the endogenous initiation codon, but is not limited thereto.

The 4) and 5) methods of modifying the amino acid sequence or the polynucleotide sequence of the polypeptide may be achieved by inducing a modification on the sequence through deletion, insertion, non-conservative or conservative substitution of the amino acid sequence of the polypeptide or the polynucleotide sequence encoding the polypeptide, or a combination thereof to increase the activity of the polypeptide; or by replacing the sequence with an amino acid sequence or a polynucleotide sequence modified to have a stronger activity, or an amino acid sequence or a polynucleotide sequence modified to increase the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is as described above.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of a polynucleotide encoding the polypeptide may be achieved by codon-optimization of an endogenous polynucleotide to increase the transcription or translation within a host cell, or by optimizing the codons thereof such that the optimized transcription and translation of a foreign polynucleotide can be achieved within the host cell.

The 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

The 9) method of regulating the cellular localization of the protein (polypeptide) may be achieved by targeting the protein (polypeptide) to a particular organelle or a particular space within the cell. For example, it may be achieved by targeting the protein (polypeptide) to the periplasm or cytoplasm through addition or removal of a leader sequence that functions in targeting proteins (polypeptides), but is not limited thereto.

Such enhancement of the polypeptide activity may mean that the activity or concentration of the corresponding polypeptide is increased relative to the activity or concentration of a polypeptide expressed in a wild-type microorganism or a microorganism before modification, or that the amount of products produced from the corresponding polypeptide is increased, but is not limited thereto.

### Weakening of Polypeptide Activity

As used herein, the term "weakening" of the activity of a polypeptide (e.g., includes proteins specified by the name of each enzyme) is a comprehensive concept including both reduced or no activity compared to its endogenous activity. The weakening may be used interchangeably with terms such as inactivation, deficiency, down-regulation, decrease, reduce, attenuation, *etc.*

The weakening may also include a case where the activity of a polypeptide itself is decreased or removed compared to the activity of the polypeptide originally possessed by a microorganism due to mutation of a polynucleotide encoding the polypeptide, *etc.*; a case where the overall level of intracellular polypeptide activity and/or concentration (expression level) is decreased compared to a natural strain due to the inhibition of expression of a gene of a polynucleotide encoding the polypeptide, or the inhibition of translation into the polypeptide, *etc.;* a case where the polynucleotide is not expressed at all; and/or a case where no polypeptide activity is observed even when the polynucleotide is expressed. The expression that the activity of a polypeptide is "inactivated, deficient, decreased, down-regulated, reduced or attenuated" compared to its endogenous activity means that the activity of a polypeptide is decreased compared to the activity of a particular polypeptide originally possessed by a parent strain before transformation or a non-modified microorganism.

The weakening of the activity of a polypeptide may be performed by any method known in the art, but the method is not limited thereto, and may be achieved by applying various methods well known in the art (*e.g*., Nakashima N et al., Bacterial cellular engineering by genome editing and gene silencing. Int J Mol Sci. 2014;15(2):2773-2793, Sambrook et al. Molecular Cloning 2012, *etc.*)*.*

Specifically, the weakening of the activity of a polypeptide of the present disclosure may be achieved by:
1) deleting a part or all of a gene encoding the polypeptide;
2) modifying the expression regulatory region (expression regulatory sequence) such that the expression of a gene encoding the polypeptide is decreased;
3) modifying the amino acid sequence constituting the polypeptide such that the polypeptide activity is removed or weakened (*e.g*., deletion/substitution/addition of one or more amino acids on the amino acid sequence);
4) modifying the gene sequence encoding the polypeptide such that the polypeptide activity is removed or weakened (*e.g*., deletion/substitution/addition of one or more of nucleotides on the nucleotide sequence of the polypeptide gene to encode a polypeptide that has been modified to remove or weaken the activity of the polypeptide);
5) modifying the nucleotide sequence encoding the initiation codon or 5'-UTR of a gene transcript encoding the polypeptide;
6) introducing an antisense oligonucleotide (*e.g*., antisense RNA), which binds complementary to a gene transcript encoding the polypeptide;
7) adding a sequence complementary to the Shine-Dalgarno (SD) sequence on the front end of the SD sequence of a gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of a gene sequence encoding the polypeptide;
9) regulating the cellular localization of the protein (polypeptide); or
10) a combination of two or more selected from above 1 to 9), but is not particularly limited thereto.

For example,
The 1) method of deleting a part or all of a gene encoding the polypeptide may be achieved by deleting all of the polynucleotide encoding the endogenous target polypeptide within the chromosome, or by replacing the polynucleotide with a polynucleotide having a partially deleted nucleotide, or with a marker gene.

The 2) method of modifying the expression regulatory region (expression regulatory sequence) may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome-binding site, and a sequence for regulating the termination of transcription and translation, but is not limited thereto.

The 3) and 4) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide, or by replacing the sequence with an amino acid sequence or a polynucleotide sequence modified to have a weaker activity, or an amino acid sequence or a polynucleotide sequence modified to have no activity, but are not limited thereto. For example, the expression of the gene may be inhibited or weakened by introducing a mutation into the polynucleotide sequence to form a termination codon, but is not limited thereto.

The 5) method of modifying the nucleotide sequence encoding the initiation codon or 5'-UTR of a gene transcript encoding the polypeptide may be achieved, for example, by substituting the nucleotide sequence with a nucleotide sequence encoding another initiation codon having a lower polypeptide expression rate than the endogenous initiation codon, but is not limited thereto.

The 6) method of introducing an antisense oligonucleotide (*e.g.*, antisense RNA), which binds complementary to a gene transcript encoding the polypeptide, can be found in the literature [Weintraub, H. et al., Antisense-RNA as a molecular tool for genetic analysis, Reviews - Trends in Genetics, Vol. 1(1) 1986].

The 7) method of adding a sequence complementary to the Shine-Dalgarno (SD) sequence on the front end of the SD sequence of a gene encoding the polypeptide to form a secondary structure, thereby inhibiting the ribosome attachment may be achieved by inhibiting mRNA translation or reducing the speed thereof.

The 8) reverse transcription engineering (RTE), which adds a promoter, which is to be reversely transcribed, on the 3' terminus of the open reading frame (ORF) of a gene sequence encoding the polypeptide, may be achieved by forming an antisense nucleotide complementary to the gene transcript encoding the polypeptide to weaken the activity.

Further, the 9) method of regulating the cellular localization of the protein (polypeptide) may be achieved by targeting the protein (polypeptide) to a particular organelle or a particular space within the cell. For example, it may be achieved by targeting the protein (polypeptide) to the periplasm or cytoplasm through addition or removal of a leader sequence that functions in targeting protein (polypeptide), but is not limited thereto.

Such weakening of the activity of the polypeptide may mean that the activity or concentration of the corresponding polypeptide is weakened relative to the activity or concentration of a polypeptide expressed in a wild-type strain or a microbial strain before modification, or that the amount of products produced from the corresponding polypeptide is decreased, but is not limited thereto.

The modification of a part or all of the polynucleotide in the microorganism of the present disclosure (a) may be achieved by homologous recombination using a vector for chromosomal insertion in the microorganism or genome editing using an engineered nuclease (*e.g*., CRISPR-Cas9), and/or (b) may be induced by light, such as ultraviolet rays and radiation, *etc.* and/or treatments of chemical substances, but is not limited thereto.

### Cultivation

As used herein, the term "cultivation" means that microorganisms are grown under appropriately controlled environmental conditions. The cultivation process of the present disclosure may be performed in a suitable culture medium and culture conditions known in the art. Such a cultivation process may be easily adjusted for use by those skilled in the art according to the microorganism to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of microorganisms as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, *etc.,* while adjusting temperature, pH, *etc*. For example, the culture medium for the microorganism of the genus *Corynebacterium* can be found in the literature ["Manual of Methods for General Bacteriology" by the American Society for Bacteriology (Washington D.C., USA, 1981)].

In the present disclosure, the carbon source may include carbohydrates, such as glucose, saccharose, lactose, fructose, sucrose, maltose, *etc.*; sugar alcohols, such as mannitol, sorbitol, *etc.*; organic acids, such as pyruvic acid, lactic acid, citric acid, *etc.*; and amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e.*, molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc*.; and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors, *etc.,* may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc*. during the cultivation of the microorganism of the present disclosure in an appropriate manner. In addition, bubble formation may be prevented during the cultivation using an antifoaming agent such as fatty acid polyglycol ester. Further, oxygen gas or a gas containing oxygen may be injected to the medium in order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature during the cultivation of the present disclosure may be in the range from 20°C to 45°C, specifically from 25°C to 40°C, and the cultivation may be continued for about 10 hours to 160 hours, but is not limited thereto.

As used herein, the term "culture" means a culture solution, a concentrated culture solution, a dried product of a culture solution, a culture filtrate, a concentrated culture filtrate, or a dried product of a culture filtrate obtained by culturing a specific microorganism in a culture medium, and means that the culture solution may include the specific microorganism while the culture filtrate does not substantially include the specific microorganism (in particular, it substantially means excluding a specific microorganism isolated by filtration, *etc.,* but does not mean that the microorganism is completely excluded from the filtrate). The formulation of the culture is not limited, and may be, for example, a liquid, emulsion, or solid.

As used herein, the term "fermentation" means that strains are not putrefactive during the process of decomposing organic matter using their own enzymes. Fermentation reaction and decay reaction proceed by similar processes, but when decomposition produces useful substances, it is called fermentation, and when odorous or harmful substances are produced, it is called decay.

In the present disclosure, the method for obtaining a fermented product from the microorganism is not particularly limited, and may be obtained according to a method commonly used in the art or similar fields.

As used herein, the term "fermented product" may include not only the fermented material itself, but also all kinds of materials including fermented products produced from the strain, such as a culture medium of the strain in which the strain and the culture coexist, a fermented product produced from the culture medium, a fermented product obtained by filtering the microorganism from the culture medium, a fermented product obtained by sterilizing the microorganism from the culture medium and filtering the same, an extract obtained by extracting the fermented product or a culture medium containing the same, a diluted solution obtained by diluting the fermented product or an extract thereof, a concentrated solution, a dried product obtained by drying the fermented product or an extract thereof, and a lysate obtained by collecting and lysing the cells of the microorganisms, *etc.*

### Detailed Description of the Present Disclosure

Hereinafter, the embodiments of the present disclosure will be described in detail as follows:
One aspect of the present disclosure provides a microorganism, in which the activity of pyruvate dehydrogenase complex subunit E1 is increased compared to the endogenous activity thereof.

In one example of the present disclosure, the microorganism of the present disclosure may have the ability to produce O-acetylhomoserine or a derivative thereof.

The increased activity of pyruvate dehydrogenase complex subunit E1 may be defined as an increased ability to produce O-acetylhomoserine or a derivative thereof of the microorganism of the present disclosure, as compared to the production ability of a natural wild-type microorganism or a non-modified microorganism (*e.g.*, a microorganism expressing a polypeptide having the activity of pyruvate dehydrogenase complex subunit E1 (*e.g*., a polypeptide of SEQ ID NO: 1), or a microorganism, in which the activity of pyruvate dehydrogenase complex subunit E1 is not increased compared to the endogenous activity thereof, or before the increase in the activity, but is not limited thereto.

For example, the activity of pyruvate dehydrogenase complex subunit E1 is determined by measuring the production ability or yield of O-acetylhomoserine or a derivative thereof, but is not limited thereto.

As used herein, the term "pyruvate dehydrogenase complex subunit E1", which is a component of the pyruvate dehydrogenase complex, may be a polypeptide including an E1 active site which converts pyruvic acid to acetyl-CoA and CO₂ in the pyruvate dehydrogenase.

The pyruvate dehydrogenase complex subunit E1 of the present disclosure may be used interchangeably with "AceE". The pyruvate dehydrogenase complex subunit E1 is known in the art, and may be specifically encoded by *aceE*, but is not limited thereto. The amino acid and polynucleotide sequences of the pyruvate dehydrogenase complex subunit E1 can be obtained from a known database, NCBI's GenBank, *etc.,* but is not limited thereto.

In one example, the pyruvate dehydrogenase complex subunit E1 may include an amino acid sequence of SEQ ID NO: 1, or an amino acid sequence having 60% or more homology or identity thereto, but is not limited as long as it has the same activity as the pyruvate dehydrogenase complex subunit E1. Specifically, any protein having the amino acid sequence of SEQ ID NO: 1, in which part of the sequence is deleted, modified, substituted, or added, may fall within the scope of the pyruvate dehydrogenase complex subunit E1 as long as it exhibits an efficacy corresponding to that of the pyruvate dehydrogenase complex subunit E1. Additionally, any protein which has or includes the amino acid sequence of SEQ ID NO: 1 or an amino acid sequence having at least 60%, 70%, 80%, 85%, 90%, 95%, 96%, 97%, 98%, or 99% or more homology or identity to SEQ ID NO: 1, which consists of the amino acid sequences above, or which essentially consists of the amino acid sequences above, and exhibits an efficacy corresponding to that of the pyruvate dehydrogenase complex subunit E1 may fall within the scope of the pyruvate dehydrogenase complex subunit E1. In one example, the pyruvate dehydrogenase complex subunit E1 may be a foreign protein, or a protein endogenously present in a microorganism of the genus *Corynebacterium* or *Corynebacterium glutamicum,* but is not limited thereto. Specifically, it may be pyruvate dehydrogenase complex subunit E1 consisting of the amino acid sequence of SEQ ID NO: 1, which is endogenously present in a microorganism of the genus *Corynebacterium* or *Corynebacterium glutamicum,* but is not limited thereto. Example thereof may include NCBI Reference No. WP_011014985.1, *etc.*

Additionally, the polynucleotide encoding the pyruvate dehydrogenase complex subunit E1 having the amino acid sequence of SEQ ID NO: 1 or an amino acid sequence having 60% or more homology or identity may be prepared, for example, based on the codon information known in the art. In one example, the protein may be encoded by a polynucleotide which may have or include the nucleotide sequence of SEQ ID NO: 2 or SEQ ID NO: 3, or a nucleotide sequence having 60% or more, 70% or more, 75% or more, 80% or more, 85% or more, 90% or more, 95% or more, 96% or more, 97% or more, 98% or more, 99% or more, and less than 100% homology or identity to the nucleotide sequence of SEQ ID NO: 2 or SEQ ID NO: 3, which may consist of the nucleotide sequence above, or which may essentially consist of the nucleotide sequence above, but is not limited thereto. Further, the nucleotide sequence of SEQ ID NO: 2 or SEQ ID NO: 3 can be obtained from a known database, NCBI's GenBank, *etc.,* but is not limited thereto.

In the present disclosure, the gene including a nucleotide sequence represented by a particular sequence number may be used interchangeably with a polynucleotide including the nucleotide sequence represented by a particular sequence number.

The polynucleotide of the present disclosure may undergo various modifications in the coding region without changing the amino acid sequence of the pyruvate dehydrogenase complex subunit E1 of the present disclosure, due to codon degeneracy or in consideration of the codons preferred in an organism in which the pyruvate dehydrogenase complex subunit E1 of the present disclosure is to be expressed. Therefore, based on codon degeneracy, it is apparent that polynucleotides which may be translated into polypeptides consisting of the amino acid sequence of the pyruvate dehydrogenase complex subunit E1 of the present disclosure or polypeptides having a homology or identity thereto may also be included within the scope of the polynucleotide of the present disclosure. For example, the polynucleotide of the present disclosure may be SEQ ID NO: 2, SEQ ID NO: 3, or a degenerated sequence thereof.

In another example, the polynucleotide of the present disclosure may have or include a nucleotide sequence having 60% or more, 70% or more, 75% or more, 80% or more, 85% or more, 90% or more, 95% or more, 96% or more, 97% or more, 98% or more, and less than 100% homology or identity to SEQ ID NO: 2 or SEQ ID NO: 3, or may consist or essentially consist of a nucleotide sequence having 60% or more, 70% or more, 75% or more, 80% or more, 85% or more, 90% or more, 95% or more, 96% or more, 97% or more, 98% or more, and less than 100% homology or identity to SEQ ID NO: 2 or SEQ ID NO: 3, but is not limited thereto.

Additionally, the polynucleotide of the present disclosure may include a probe that may be prepared from a known gene sequence, for example, any sequence which may hybridize with a sequence complementary to all or part of the polynucleotide sequence of the present disclosure under stringent conditions to encode the pyruvate dehydrogenase complex subunit E1 of the present disclosure without limitation.

As used herein, the term "O-acetylhomoserine or a derivative thereof" includes O-acetylhomoserine and products that can be produced using O-acetylhomoserine as a precursor. In one specific example, the O-acetylhomoserine or a derivative thereof may be any one or more selected from O-acetylhomoserine, L-methionine, and L-homoserine.

For the purpose of the present disclosure, the microorganism of the present disclosure may include all microorganisms capable of producing the desired O-acetylhomoserine or a derivative thereof by increasing the activity of pyruvate dehydrogenase complex subunit E1 as compared to the endogenous activity thereof. For example, the microorganism of the present disclosure is characterized in that the activity of pyruvate dehydrogenase complex subunit E1 is increased as compared to the endogenous activity thereof, thereby increasing the ability to produce O-acetylhomoserine or a derivative thereof, and it may be a genetically modified microorganism or a recombinant microorganism, but is not limited thereto. Specifically, the recombinant microorganism having an increased ability to produce O-acetylhomoserine or a derivative thereof may be a microorganism having an increased ability to produce O-acetylhomoserine as compared to a natural wild-type microorganism, or a non-modified microorganism having an endogenous activity of pyruvate dehydrogenase complex subunit E1, but is not limited thereto.

In one example, the microorganism having the ability to produce O-acetylhomoserine or a derivative thereof, which is a prokaryotic or eukaryotic microorganism capable of producing O-acetylhomoserine or a derivative thereof in an organism, may include all of microorganisms that endogenously have the ability to produce O-acetylhomoserine or a derivative thereof, or microorganisms, in which the ability to produce O-acetylhomoserine or a derivative thereof has been imparted to a microorganism not endogenously having the ability to produce O-acetylhomoserine or a derivative thereof. The ability to produce O-acetylhomoserine or a derivative thereof may be imparted or enhanced by the activity of pyruvate dehydrogenase complex subunit E1 or improvement of species.

In one example, the recombinant microorganism having the ability to produce O-acetylhomoserine or a derivative thereof may include all microorganisms, which may be transformed through a vector and thus capable of producing O-acetylhomoserine or a derivative thereof by increasing the activity of pyruvate dehydrogenase complex subunit E1.

For example, the microorganism for producing O-acetylhomoserine or a derivative thereof may be a microorganism which endogenously includes a protein consisting of the amino acid sequence of SEQ ID NO: 1, or a protein consisting of an amino acid sequence having at 60%, 70%, 75%, 80%, 85%, 90%, 95%, 96%, 97%, 98%, 99%, 99.5%, 99.7% or 99.9% or more homology or identity to SEQ ID NO: 1.

For example, the microorganism for producing O-acetylhomoserine or a derivative thereof may be a microorganism which endogenously includes a polynucleotide sequence capable of encoding a protein including an amino acid sequence having at least 60% homology to SEQ ID NO: 1; or a polynucleotide including a nucleotide sequence of SEQ ID NO: 2 or SEQ ID NO: 3, or a nucleotide sequence having 60% or more, 70% or more, 75% or more, 80% or more, 85% or more, 90% or more, 95% or more, 96% or more, 97% or more, or 98% or more homology or identity to the nucleotide sequence of SEQ ID NO: 2 or SEQ ID NO: 3.

The microorganism of the present disclosure may include all microorganisms in which the activity of pyruvate dehydrogenase complex subunit E1 is increased as compared to the endogenous activity thereof by various known methods.

In one embodiment, the microorganism, in which the activity of pyruvate dehydrogenase complex subunit E1 is increased as compared to the endogenous activity thereof, may be a microorganism having an increased activity by substituting the sequence regulating the expression of the sequence encoding the pyruvate dehydrogenase complex subunit E1 with another sequence, modifying the nucleotide sequence encoding the pyruvate dehydrogenase complex subunit E1, or substituting the initiation codon, thereby increasing the expression of the protein, but is not limited thereto. In one embodiment, the microorganism, in which the activity of pyruvate dehydrogenase complex subunit E1 is increased as compared to the endogenous activity thereof, may be a microorganism having an increased activity by substituting the sequence regulating the expression of the sequence encoding the pyruvate dehydrogenase complex subunit E1 with a sequence having a stronger activity as compared to the endogenous expression regulatory sequence, thereby increasing the expression of the protein, but is not limited thereto.

In one example, the microorganism having an increased ability to produce O-acetylhomoserine or a derivative thereof of the present disclosure may be a microorganism having an increased ability to produce O-acetylhomoserine or a derivative thereof compared to a non-modified microorganism, but is not limited thereto. In one example, the non-modified microorganism that is the target strain for comparing the increase in the ability to produce O-acetylhomoserine or a derivative thereof may be ATCC13032 strain, but is not limited thereto.

In one example, the microorganism having an increased ability to produce O-acetylhomoserine or a derivative thereof may have an increased ability to produce O-acetylhomoserine or a derivative thereof by about 1% or more, specifically, about 1% or more, about 2.5% or more, or about 5% or more, as compared to the ability to produce O-acetylhomoserine or a derivative thereof of a parent microorganism (parent strain) before modification or a non-modified microorganism, but is not limited thereto, as long as it has an increased + value compared to the production ability of a parent microorganism (parent strain) before modification or a non-modified microorganism. In another example, the recombinant microorganism having an increased ability to produce O-acetylhomoserine or a derivative thereof may have an increased ability to produce O-acetylhomoserine or a derivative thereof by about 1.01 times or more, about 1.02 times or more, about 1.03 times or more, about 1.04 times or more, about 1.05 times or more, about 1.06 times or more, about 1.07 times or more, about 1.08 times or more, about 1.09 times or more, or about 1.1 times or more, as compared to that of a parent microorganism (parent strain) before modification or a non-modified microorganism, but is not limited thereto.

In one example, the microorganism having the ability to produce O-acetylhomoserine or a derivative thereof may be a prokaryotic cell or eukaryotic cell, but may specifically be a prokaryotic cell. The prokaryotic cell may include, for example, a microbial strain of the genus *Escherichia*, the genus *Erwinia*, the genus *Serratia*, the genus *Providencia,* the genus *Corynebacterium,* the genus *Pseudomonas*, the genus *Leptospira*, the genus *Salmonella*, the genus *Brevibacteria*, the genus *Hypomononas*, the genus *Chromobacterium*, and the genus *Norcardia*, or fungi or yeasts, but is not limited thereto. Specifically, it may be a microbial strain of the genus *Escherichia*, the genus *Corynebacterium,* the genus *Leptospira*, and yeasts. More specifically, it may be a microorganism of the genus *Corynebacterium.*

As the microorganism according to any one of the above-described embodiments, the microorganism of the present disclosure may be a microorganism of the genus *Corynebacterium.*

In one example, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli*, *Corynebacterium imitans, Corynebacterium testudinoris,* or *Corynebacterium flavescens.* Specifically, the microorganism of the present disclosure may be a microorganism of the genus *Corynebacterium,* more specifically, *Corynebacterium glutamicum,* but is not limited thereto.

Additionally, the microorganism of the genus *Corynebacterium* having the ability to produce O-acetylhomoserine or a derivative thereof of the present disclosure may include all of the following: a natural wild-type microorganism itself; a microorganism of the genus *Corynebacterium* in which the activity of a gene associated with the production mechanism of O-acetylhomoserine or a derivative thereof is increased or decreased, thus having an enhanced ability to produce O-acetylhomoserine or a derivative thereof; or a microorganism of the genus *Corynebacterium* in which the activity of a foreign gene is introduced or increased, thus having an enhanced ability to produce O-acetylhomoserine or a derivative thereof.

The microorganism having the ability to produce O-acetylhomoserine or a derivative thereof of the present disclosure may additionally include a modification that increases the ability to produce the desired O-acetylhomoserine or a derivative thereof.

In one example, the microorganism of the present disclosure may be a microorganism in which the activity of O-acetylhomoserine transferase (MetX) is increased compared to the endogenous activity thereof.

In another example, the microorganism of the present disclosure may include a modification of the inner membrane protein YjeH. The contents of WO2021-125896 A1 may be incorporated herein by reference.

In still another example, the microorganism of the present disclosure may include a modification in which the expression of aspartokinase (LysC) is enhanced and/or feedback inhibition is eliminated. In this regard, the contents of US 10662450 B2 may be incorporated herein by reference.

In still another example, the microorganism of the present disclosure may be a microorganism in which the activity of cystathionine gamma-synthase is weakened. For example, the microorganism of the present disclosure may be a microorganism in which the activity of the *metB* gene encoding cystathionine gamma-synthase is weakened. For example, the microorganism may be a microorganism in which all or part of the *metB* gene is deleted.

In still another example, the microorganism of the present disclosure may be a microorganism in which the activity of O-acetylhomoserine (thiol)-lyase is weakened. For example, the microorganism of the present disclosure may be a microorganism in which the activity of the *metY* gene encoding O-acetylhomoserine (thiol)-lyase is weakened. For example, the microorganism may be a microorganism in which all or part of the *metY* gene is deleted.

In still another example, the microorganism of the present disclosure may be a microorganism in which the activity of the endogenous protein Ncgl0616 is weakened. For example, the microorganism may be a microorganism in which all or part of the gene encoding Ncgl0616 is deleted.

Another aspect of the present disclosure provides a method for producing O-acetylhomoserine or a derivative thereof, including: culturing a microorganism, in which the activity of pyruvate dehydrogenase complex subunit E1 is increased compared to the endogenous activity thereof, in a medium.

In the method of the present disclosure, the cultivation of the microorganism may be performed using any culture conditions and cultivation methods known in the art. Such a cultivation process may be easily adjusted for use by those skilled in the art according to the strain to be selected.

The O-acetylhomoserine or a derivative thereof produced by the cultivation of the present disclosure may be released into the medium or remain in the cells.

In one embodiment, the method for producing O-acetylhomoserine or a derivative thereof of the present disclosure may further include a step of preparing the microorganism of the present disclosure, a step of preparing a medium for culturing the strain, or a combination thereof (regardless of the order, in any order), for example, prior to the culturing step.

The method for producing O-acetylhomoserine or a derivative thereof of the present disclosure may further include a step of recovering the desired substance, specifically, O-acetylhomoserine or a derivative thereof, from the cultured microorganism, a culture of the microorganism, a fermented product of the microorganism, or the culture medium. The recovering step may be further included after the culturing step.

In the recovering step, the desired O-acetylhomoserine or a derivative thereof may be collected using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC or a combination thereof may be used, and the desired substance, specifically, O-acetylhomoserine or a derivative thereof, can be recovered from the medium or the microorganisms using suitable methods known in the art.

Further, the method for producing O-acetylhomoserine or a derivative thereof of the present disclosure may further include a purification step, which may be performed using an appropriate method known in the art. In one example, when the method for producing O-acetylhomoserine or a derivative thereof of the present disclosure includes both a recovering step and a purification step, the recovering step and the purification step may be performed continuously or intermittently regardless of the order or simultaneously, or may be integrated into one step, but the method is not limited thereto.

In the method of the present disclosure, the increase in the activity of pyruvate dehydrogenase complex subunit E1, and O-acetylhomoserine or a derivative thereof, *etc.* are as described in other aspects above.

Still another aspect of the present disclosure provides a composition for producing O-acetylhomoserine or a derivative thereof, including: a microorganism, in which the activity of pyruvate dehydrogenase complex subunit E1 is increased compared to the endogenous activity thereof; a culture of the microorganism; a fermented product of the microorganism; or a combination of two or more thereof.

The composition of the present disclosure may further include any suitable excipient commonly used in compositions for producing O-acetylhomoserine or a derivative thereof, and such excipients may include, for example, preservatives, wetting agents, dispersing agents, suspending agents, buffers, stabilizers, or isotonic agents, *etc*., but are not limited thereto.

In one embodiment, each component present in the composition of the present disclosure may be contained in a microbiologically effective amount, or in an amount that can be appropriately present in the composition for production.

In the composition of the present disclosure, the increase in the activity of pyruvate dehydrogenase complex subunit E1, and O-acetylhomoserine or a derivative thereof, *etc.* are as described in other aspects above.

Still another aspect of the present disclosure provides use of a microorganism, in which the activity of pyruvate dehydrogenase complex subunit E1 is increased compared to the endogenous activity thereof, for the production of O-acetylhomoserine or a derivative thereof.

In the use of the present disclosure, the increase in the activity of pyruvate dehydrogenase complex subunit E1, and O-acetylhomoserine or a derivative thereof, *etc.* are as described in other aspects above.

Still another aspect of the present disclosure provides a method for preparing a microorganism for producing O-acetylhomoserine or a derivative thereof, including modifying the microorganism to have an increased activity of pyruvate dehydrogenase complex subunit E1 compared to the endogenous activity thereof

In the method for preparing a microorganism of the present disclosure, the increase in the activity of pyruvate dehydrogenase complex subunit E1, and O-acetylhomoserine or a derivative thereof, *etc.* are as described in other aspects above.

### [Mode for Carrying Out the Invention]

### Example 1. Preparation of Strains for Producing O-Acetylhomoserine or Homoserine

Strains were prepared to be used in evaluating the ability to produce O-acetylhomoserine and homoserine.

### Example 1-1. Deletion of metB

The *metB* gene encoding cystathionine γ-synthase involved in a degradation pathway of O-acetylhomoserine was obtained via PCR using chromosomal DNA of *Corynebacterium glutamicum* ATCC13032 as a template. Information on a nucleotide sequence of the *metB* gene (NCBI No. NcgI2360, SEQ ID NO: 4) was obtained from GenBank of the National Institutes of Health (USA), and primers (SEQ ID NOS: 6 and 7) including the N-terminus of the *metB* gene and a linker and primers (SEQ ID NOS: 8 and 9) including the C-terminus and the linker were synthesized based thereon. The primer sequences are shown in Table 1 below.

**[Table 1]**

| SEQ ID NO: | Name of Sequence | Sequences |
|---|---|---|
| 6 | metB_N_del F | GGCTTCGGAGTTGGAGCG |
| 7 | metB_N_del R | GCCAAATAGTTTcccgggGGTAGATCAACTCCTGTAAT CA |
| 8 | metB_C_del F | CAGGAGTTGATCTACCcccgggAAACTATTTGGCGGC AAG |
| 9 | metB_C_del R | TCGCGTCTGGGTGCGCATC |

PCR was performed based on the chromosomal DNA of ATCC13032 as a template using the primers of SEQ ID NOS: 6 and 7 and SEQ ID NOS: 8 and 9. PfuUltra^{™} high-fidelity DNA polymerase (Stratagene) was used as a polymerase, and PCR was performed by repeating 30 cycles of denaturation at 96°C for 30 seconds, annealing at 53°C for 30 seconds, and polymerization at 72°C for 1 minute. As a result, an amplified gene (558 bp) containing the N-terminus of the *metB* gene and the linker and an amplified gene (527 bp) containing the C-terminus of the *metB* gene and the linker were obtained, respectively. PCR was performed based on the two amplified genes obtained as described above as templates by repeating 10 cycles of denaturation at 96°C for 60 seconds, annealing at 50°C for 60 seconds, and polymerization at 72°C for 1 minute, and then adding the primers of SEQ ID NOS: 6 and 9 thereto, followed by repeating 20 cycles of polymerization. As a result, an inactivation cassette (1064 bp) including the N-terminus-linker-C-terminus of the *metB* gene was obtained.

The pDCM2 vector was treated with the restriction enzyme Smal, and the obtained PCR product (1064 bp) was fusion-cloned with the pDCM2 vector treated with the restriction enzyme Smal using an In-Fusion^{®} HD Cloning Kit (Clontech). The cloned vector was transformed with *E. coli* DH5α, and the transformed *E. coli* was smeared on an LB solid medium containing kanamycin (25 mg/L). Colonies transformed with the plasmid on the LB medium were selected, and the plasmid was obtained using a plasmid extraction method (US5981235 A). Finally, pDCM2-ΔmetB recombinant vector cloned with the *metB* gene deletion cassette was prepared.

The thus-prepared pDCM2-ΔmetB vector was transformed with the ATCC13032 ΔNCgI2335::PCJ7-yjeH(eco,F351L) (KCCM12634P; WO2021-125896 A1) strain using an electric-pulse method and subjected to a secondary crossover process to obtain ATCC13032 ΔNCgI2335::PCJ7-yjeH(eco,F351L) ΔmetB in which the *metB* gene was inactivated in the chromosome. The inactivation of the *metB* gene was ultimately confirmed by comparison with ATCC13032 in which the *metB* gene was not inactivated after performing PCR using the primers of SEQ ID NOS: 6 and 9.

### Example 1-2. Deletion of metY

The *metY* gene encoding O-acetylhomoserine (thiol)-lyase involved in a degradation pathway of O-acetylhomoserine was obtained via PCR using chromosomal DNA of *Corynebacterium glutamicum* ATCC13032 as a template. Information on a nucleotide sequence of the *metY* gene (NCBI No. Ncgl0625, SEQ ID NO: 19) was obtained from GenBank of the National Institutes of Health (USA), and primers (SEQ ID NOS: 20 and 21) including the N-terminus of the *metY* gene and a linker and primers (SEQ ID NOS: 22 and 23) including the C-terminus and the linker were synthesized based thereon. The primer sequences are shown in Table 2 below.

**[Table 2]**

| SEQ ID NO: | Name of Sequence | Sequences |
|---|---|---|
| 20 | metY_N_del F | CCAAAGACAAGGAGACCA |
| 21 | metY_N_del R | AGTCTATTTAAAGcccgggTTGGAGGTCCTTAAGAGTTTT |
| 22 | metY_C_del F | TAAGGACCTCCAAcccgggCTTTAAATAGACTCACCCCAG |
| 23 | metY_C_del R | ATGCCGAGTGCGTCGAGG |

PCR was performed based on the chromosomal DNA of ATCC13032 as a template using the primers of SEQ ID NOS: 20 and 21 and SEQ ID NOS: 22 and 23. PfuUltra^{™} high-reliability DNA polymerase (Stratagene) was used as a polymerase, and PCR was performed by repeating 30 cycles of denaturation at 96°C for 30 seconds, annealing at 53°C for 30 seconds, and polymerization at 72°C for 1 minute. As a result, an amplified gene (548 bp) containing the N-terminus of the *metY* gene and the linker and an amplified gene (550 bp) containing the C-terminus of the *metY* gene and the linker were obtained, respectively. PCR was performed based on the two amplified genes obtained as described above as templates by repeating 10 cycles of denaturation at 96°C for 60 seconds, annealing at 50°C for 60 seconds, and polymerization at 72°C for 1 minute, and then adding the primers of SEQ ID NOS: 20 and 23 thereto, followed by repeating 20 cycles of polymerization. As a result, an inactivation cassette (1077 bp) including the N-terminus-linker-C-terminus of the *metY* gene was obtained.

The pDCM2 vector was treated with the restriction enzyme Smal, and the obtained PCR product (1077 bp) was fusion-cloned with the pDCM2 vector treated with the restriction enzyme Smal using an In-Fusion^{®} HD Cloning Kit (Clontech). The cloned vector was transformed with *E. coli* DH5α, and the transformed *E. coli* was smeared on an LB solid medium containing kanamycin (25 mg/L). Colonies transformed with the plasmid on the LB medium were selected, and the plasmid was obtained using a plasmid extraction method (US5981235 A). Finally, pDCM2-ΔmetY recombinant vector cloned with the *metY* gene deletion cassette was prepared.

The thus-prepared pDCM2-ΔmetY vector was transformed with the ATCC13032 ΔNCgI2335::PCJ7-yjeH(eco,F351L) ΔmetB using an electric-pulse method and subjected to a secondary crossover process to obtain ATCC13032 ΔNCgI2335::PCJ7-yjeH(eco,F351L) ΔmetB ΔmetY in which the *metY* gene was additionally inactivated in the chromosome.

The inactivation of the *metY* gene was ultimately confirmed by comparison with ATCC13032 in which the *metY* gene was not inactivated after performing PCR using the primers of SEQ ID NOS: 20 and 23.

### Example 1-3. Introduction of lysC(L377K)

In order to introduce a mutation(L377K) (US 10662450 B2) for enhancement of expression of the *lysC* gene and relief of feedback inhibition by L-lysine and L-threonine (US 10662450 B2) to the *Corynebacterium glutamicum* ATCC13032-derived *lysC* gene (SEQ ID NO: 24) encoding aspartokinase and to prepare a vector including the variant *lysC* gene, a pair of primers (SEQ ID NOS: 25 and 26) for amplifying a 5' upstream region and a pair of primers (SEQ ID NOS: 27 and 28) for amplifying a 3' downstream region with respect to the mutation position were designed. The primer sequences are shown in Table 3 below.

**[Table 3]**

| SEQ ID NO: | Name of Sequence | Sequences |
|---|---|---|
| 25 | lysC_L377K_5 F | CAGAAGCTGGAAAAGCTCA |
| 26 | lysC_L377K_5 R | ATCTCAGAGGTGGAAATCttTTCGATGTTCACGTTGAC AT |
| 27 | lysC_L377K_3 F | TGTCAACGTGAACATCGAAaaGATTTCCACCTCTGAG ATT |
| 28 | lysC_L377K_3 R | TCCTTGTCGGAAGGGTTCA |

PCR was performed based on the chromosome of *Corynebacterium glutamicum* ATCC13032 as a template using the primers of SEQ ID NOS: 25 and 26 and SEQ ID NOS: 27 and 28. PCR was performed under the following conditions: after denaturation at 95°C for 5 minutes, 30 cycles of denaturation at 95°C for 30 seconds, annealing at 55°C for 30 seconds, and polymerization at 72°C for 30 seconds were repeated, and then polymerization was performed at 72°C for 7 minutes. As a result, a 512 bp DNA fragment of a 5' upstream region and a 522 bp DNA fragment of a 3' downstream region with respect to the mutation of the *lysC* gene were obtained. Thereafter, PCR was performed based on the two amplified DNA fragments as templates using the primers of SEQ ID NOS: 25 and 28. PCR was performed under the following conditions: after denaturation at 95°C for 5 minutes, 30 cycles of denaturation at 95°C for 30 seconds, annealing at 55°C for 30 seconds, and polymerization at 72°C for 30 seconds were repeated, and then polymerization was performed at 72°C for 7 minutes. As a result, a 1011 bp DNA fragment including the variant lysC (L377K) gene encoding an aspartokinase variant, in which leucine at the 377th position was substituted with lysine, was amplified.

The pDCM2 vector was treated with the restriction enzyme Smal, and the obtained PCR product (1011 bp) was fusion-cloned with the pDCM2 vector treated with the restriction enzyme Smal using an In-Fusion^{®} HD Cloning Kit (Clontech). The cloned vector was transformed with *E. coli* DH5α, and the transformed *E. coli* was smeared on an LB solid medium containing kanamycin (25 mg/L). Colonies transformed with the plasmid on the LB medium were selected, and the plasmid was obtained using a plasmid extraction method (US5981235 A). Finally, pDCM2- lysC(L377K) recombinant vector cloned with the cassette including *lysC* (L377K) gene substitution was prepared.

The thus-prepared pDCM2-lysC(L377K) vector was transformed with the ATCC13032 ΔNCgI2335::PCJ7-yjeH(eco,F351L) ΔmetB ΔmetY using an electric-pulse method and subjected to a secondary crossover process to obtain *Corynebacterium glutamicum* ATCC13032 ΔNCgI2335::PCJ7-yjeH(eco,F351L) ΔmetB ΔmetY lysC(L377K) in which a nucleotide mutation was introduced within the *lysC* gene on the chromosome. The introduction of the nucleotide mutation was ultimately identified by PCR performed using the primers of SEQ ID NOS: 25 and 28 and then comparing the sequence with that of the wild-type *lysC* gene by sequencing.

### Example 1-4. Deletion of NCgI0616

A vector was prepared to delete the endogenous gene NCgI0616 (SEQ ID NO: 29) in the *Corynebacterium glutamicum* ATCC13032.

Specifically, a pair of primers (SEQ ID NOS: 30 and 31) for amplifying a 5' upstream region and a pair of primers (SEQ ID NOS: 32 and 33) for amplifying a 3' downstream region with respect to the position of NCgI0616 gene of SEQ ID NO: 29 were designed. The primer sequences are shown in Table 4 below.

**[Table 4]**

| SEQ ID NO: | Name of Sequence | Sequences |
|---|---|---|
| 30 | 0616 _del up F | TCGAGCTCGGTACCCAGCATAATTCCATACTCCCAC |
| 31 | 0616 _del | GGTAGCGCTTGGGCTGCAGTACTCTCGACGGCGTGCTCAATT |
| | up R | |
| 32 | 0616 _del down F | AATTGAGCACGCCGTCGAGAGTACTGCAGCCCAAGCGCTACC |
| 33 | 0616 _del down R | CTCTAGAGGATCCCCCTACGACGCTACTGCGCAG |

PCR was performed based on the chromosome of the wild-type ATCC13032 as a template using the primers of SEQ ID NOS: 30 and 31 and SEQ ID NOS: 32 and 33. PCR was performed under the following conditions: after denaturation at 95°C for 5 minutes, 30 cycles of denaturation at 95°C for 30 seconds, annealing at 55°C for 30 seconds, and polymerization at 72°C for 30 seconds were repeated, and then polymerization was performed at 72°C for 7 minutes. As a result, a 701 bp DNA fragment of a 5' upstream region and a 699 bp DNA fragment of a 3' downstream region with respect to the deletion position of the NCgI0616 gene were obtained.

Thereafter, PCR was performed based on the two amplified DNA fragments as templates using the primers of SEQ ID NOS: 30 and 33. PCR was performed under the following conditions: after denaturation at 95°C for 5 minutes, 30 cycles of denaturation at 95°C for 30 seconds, annealing at 55°C for 30 seconds, and polymerization at 72°C for 90 seconds were repeated, and then polymerization was performed at 72°C for 7 minutes. As a result, a 1410 bp DNA fragment including the region from which the NCgI0616 gene could be deleted was amplified.

The pDCM2 vector was treated with the restriction enzyme Smal, and the obtained PCR product (2912 bp DNA fragment) was fusion-cloned with the pDCM2 vector treated with the restriction enzyme Smal using an In-Fusion^{®} HD Cloning Kit (Clontech). The cloned vector was transformed with *E. coli* DH5α, and the transformed *E*. *coli* was smeared on an LB solid medium containing kanamycin (25 mg/L). Colonies transformed with the plasmid on the LB medium were selected, and the plasmid was obtained using a plasmid extraction method (US5981235 A). Finally, pDCM2-ΔNCgI0616 recombinant vector cloned with the NCgI0616 deletion cassette was prepared.

**[Table 5]**

| SEQ ID NO: | Name of Sequence | Sequences |
|---|---|---|
| 34 | 0616 _del F | GGCTTCCAACTGTAATGG |
| 35 | 0616 _del R | TAGAACACCCAGCTAACA |

The thus-prepared pDCM2-ΔNCgI0616 vector was transformed using an electric-pulse method and subjected to a secondary crossover process to obtain ATCC13032 ΔNCgI2335::PCJ7-yjeH(eco,F351L) ΔmetB ΔmetY lysC(L377K) ΔNCgI0616 in which the NCgI0616 gene was deleted in the chromosome. The inactivation of the NCgI0616 gene was ultimately confirmed by comparison with ATCC13032 in which the NCgI0616 gene was not inactivated after performing PCR using the primers of SEQ ID NOS: 34 and 35.

### Example 2. Preparation of Plasmid for Enhancing the Activity of Pyruvate Dehydrogenase Complex Subunit E1

A vector was prepared to enhance the activity of pyruvate dehydrogenase complex subunit E1 (NCgI2167_hereinafter referred to as aceE). Specifically, to prepare a vector for enhancing the pyruvate dehydrogenase complex subunit E1 (NCgI2167, SEQ ID NO: 1), the wild-type promoter of the aceE gene (SEQ ID NO: 2) was replaced with Pcj7 using a strong promoter known as the Pcj7 promoter (US 7662943 B2) (SEQ ID NO: 3), thereby preparing a plasmid to enhance aceE activity. The upstream and downstream regions of the aceE gene were obtained. Specifically, to prepare a strain into which aceE having the Pcj7 promoter was introduced, PCR was performed based on the chromosomal DNA of *Corynebacterium glutamicum* ATCC13032 as a template to amplify the upstream region of the aceE gene using primers of SEQ ID NO: 12 and SEQ ID NO: 13, and the downstream region of the *aceE* gene using primers of SEQ ID NO: 14 and SEQ ID NO: 15, respectively. Additionally, Pcj7 promoter fragments were obtained using SEQ ID NO: 10 and SEQ ID NO: 11 based on pDCM2-Pcj7 as a template. The primer sequences used to perform each of the PCRs are as shown in Table 6 below.

**[Table 6]**

| SEQ ID NO: | Name of Sequence | Sequences |
|---|---|---|
| 10 | Pcj7-F | AGAAACATCCCAGCGCTACTAATAGGGAGCGTTG |
| 11 | Pcj7-R | ATGGGATACGTACCCAACGAAAGGAAACACTC |
| 12 | aceE -5'-F | GTGAATTCGAGCTCGGTACCCCACTGGCCAGCGATTA ACTTT |
| 13 | aceE -5'-R | TAGTAGCGCTGGGATGTTTCTTTCCACACCTCCTGTTG GAAT |
| 14 | aceE -3'-F | CCAACGAAAGGAAACACTCATGGCCGATCAAGCAAAA CTT |
| 15 | aceE -3'-R | GGTCGACTCTAGAGGATCCCCCCTGGAAGAAGATCTG GTCGC |

PfuUltra^{™} high-reliability DNA polymerase (Stratagene) was used as a polymerase for the PCR reaction, and PCR was performed by repeating 28 cycles of denaturation at 95°C for 30 seconds, annealing at 55°C for 30 seconds, and polymerization at 72°C for 1 minute.

As a result, a 318 bp DNA fragment of the Pcj7 promoter region, a 526 bp DNA fragment upstream of *Corynebacterium glutamicum* ATCC13032 aceE, and a 527 bp DNA fragment downstream thereof were obtained, respectively. PCR was performed based on the amplified promoter and DNA fragments as templates using primers of SEQ ID NO: 12 and SEQ ID NO: 15. The PCR was performed under the following conditions: after denaturation at 95°C for 5 minutes, 28 cycles of denaturation at 95°C for 30 seconds, annealing at 55°C for 30 seconds, and polymerization at 72°C for 2 minutes were repeated, and then polymerization was performed at 72°C for 5 minutes. The two fragments obtained above were subjected to DNA purification, and then fusion cloning was performed using an In-Fusion^{®} HD cloning kit (Clontech) based on the pDCM2 vector (Korean Patent No. WO2021-187781 A1) treated with Smal restriction enzyme to obtain a plasmid according to the provided manual. The resulting vector was named pDCM2-Pcj7_aceE.

### Example 3. Preparation of O-Acetylhomoserine Strain with Enhanced Activity of Pyruvate Dehydrogenase Complex Subunit E1

To determine whether the activity-enhancing strain has an effect of increasing the production ability in the *Corynebacterium glutamicum* strain having the ability to produce O-acetylhomoserine and homoserine, the pDCM2-Pcj7_aceE vector prepared in Example 2 was transformed with ATCC13032 ΔNCgI2335::PCJ7-yjeH(eco,F351L) ΔmetB ΔmetY lysC(L377K)ΔNCgl0616, which is the *Corynebacterium glutamicum* strain for producing O-acetylhomoserine and homoserine prepared in Example 1, by homologous recombination in the chromosome (van der Rest et al., Appl Microbiol Biotechnol 52:541-545, 1999).

The resulting recombinant strain was named CM04-8001 (ATCC13032 ΔNCgI2335::PCJ7-yjeH(eco,F351L) ΔmetB ΔmetY lysC(L377K)ΔNCgl0616-Pcj7_aceE).

### Example 4. Evaluation of Production Ability of O-Acetylhomoserine and Homoserine Strain with Enhanced Activity of Pyruvate Dehydrogenase Complex Subunit E1

A flask evaluation was performed to compare the production ability of Corynebacterium *glutamicum* ATCC13032 ΔNCgI2335::PCJ7-yjeH(eco,F351L) ΔmetB ΔmetY lysC(L377K)ΔNCgl0616 and CM04-8001, which are the O-acetylhomoserine and homoserine-producing strains.

One inoculation loop of each of the strains was inoculated into a 250 mL corner-baffled flask containing 25 mL of the following medium and then cultured while shaking at 33°C for 20 hours at 200 rpm. The concentrations of O-acetylhomoserine were analyzed using HPLC, and the analyzed concentrations are shown in Table 7.

### O-Acetylhomoserine Production Medium (pH 7.2)

30 g of glucose, 2 g of KH₂PO₄, 3 g of urea, 40 g of (NH₄)₂SO₄, 2.5 g of peptone, 5 g (10 mL) of corn steep liquor (CSL, Sigma), 0.5 g of MgSO₄·7H₂O, and 20 g of CaCO₃ (based on 1 L of distilled water)

**[Table 7]**

| Strains | O-AH (g/L) |
|---|---|
| ATCC13032 | 0.27 |
| ATCC13032ΔNCgI2335::PCJ7-yjeH(eco,F351L) ΔmetB ΔmetY lysC(L377K)ΔNCgI0616 | 1.73 |
| CM04-8001 (ATCC13032 ΔNCgI2335::PCJ7-yjeH(eco,F351L) ΔmetB ΔmetY lysC(L377K)ΔNCgl0616 -Pcj7_aceE) | 2.00 |

As shown in Table 7 above, it was confirmed that the concentration of O-acetylhomoserine was increased in the O-acetylhomoserine-producing strain with enhanced activity of pyruvate dehydrogenase complex subunit E1, compared to ATCC13032 and ATCC13032ΔNCgI2335::PCJ7-yjeH(eco,F351L) ΔmetB ΔmetY lysC(L377K)ΔNCgl0616.

### Example 5. Preparation of Plasmid Introduced with O-Acetylhomoserine Transferase (MetX)

In order to amplify a gene encoding O-acetylhomoserine transferase (MetX), information on a nucleotide sequence of *metX* gene (NCBI No. NCgI0624, SEQ ID NO: 16) was obtained from GenBank of the National Institutes of Health (USA), a BamHI restriction enzyme site was inserted into both ends of each of primers (SEQ ID NOS: 17 and 18) for amplification from a promoter region (located about 300 bp upstream from a start codon) to a terminator region (located about 100 bp downstream from a stop codon) based thereon. PCR was performed under the following conditions: after denaturation at 95°C for 5 minutes, 30 cycles of denaturation at 95°C for 30 seconds, annealing at 55°C for 30 seconds, and polymerization at 72°C for 90 seconds were repeated, and then polymerization was performed at 72°C for 7 minutes. As a result, a 1546 bp DNA fragment of a coding region of the *metX* gene was obtained. A pECCG117 vector (KR 10-0057684 B1) and the *metX* DNA fragment were treated with the restriction enzyme BamHI, ligated using a DNA ligase, and cloned to obtain a plasmid, which was named pECCG117-metX WT. The primer sequences are as shown in Table 8 below.

**[Table 8]**

| SEQ ID NO: | Name of Sequence | Sequences |
|---|---|---|
| SEQ ID NO: 17 | metX F | GGATCCCCTCGTTGTTCACCCAGCAACC |
| SEQ ID NO: 18 | metX R | GGATCCCAAAGTCACAACTACTTATGTTAG |

After the pECCG117-metX WT vector prepared above was introduced into ATCC13032 ΔNCgI2335::PCJ7-yjeH(eco,F351L) ΔmetB ΔmetY lysC(L377K)ΔNCgI0616 and CM04-8001 using an electric-pulse method, the strains were smeared on a selective medium containing kanamycin (25 mg/L) and cultured to obtain respective transformants.

For comparison of the O-acetylhomoserine-producing ability of the strains prepared above, the strains were cultured by way of the following method, and O-acetylhomoserine contained in the culture solutions was analyzed.

One inoculation loop of each of the strains was inoculated to a 250 mL corner-baffled flask containing 25 mL of the following medium and then cultured while shaking at 33°C for 20 hours at 200 rpm. The concentrations of O-acetylhomoserine were analyzed using HPLC, and the analyzed concentrations are shown in Table 9.

### O-Acetylhomoserine Production Medium (pH 7.2)

30 g of glucose, 2 g of KH₂PO₄, 3 g of urea, 40 g of (NH₄)₂SO₄, 2.5 g of peptone, 5 g (10 mL) of corn steep liquor (CSL, Sigma), 0.5 g of MgSO₄·7H₂O, 400 mg of methionine, and 20 g of CaCO₃ (based on 1 L of distilled water)

**[Table 9]**

| Strains | O-AH (g/L) |
|---|---|
| ATCC13032ΔNCgI2335::PCJ7-yjeH(eco,F351L) ΔmetB ΔmetY lysC(L377K)ΔNCgl0616 | 1.73 |
| CM04-8001 (ATCC13032 ΔNCgI2335::PCJ7-yjeH(eco,F351L) ΔmetB ΔmetY lysC(L377K)ΔNCgl0616 -Pcj7_aceE) | 2.00 |
| ATCC13032 ΔNCgI2335::PCJ7-yjeH(eco,F351L) ΔmetB ΔmetY lysC(L377K)ΔNCgI0616) **/pECCG117-metX WT** | 2.83 |
| CM04-8001 (ATCC13032 ΔNCgI2335::PCJ7-yjeH(eco,F351L) ΔmetB ΔmetY lysC(L377K)ΔNCgI0616 -Pcj7_aceE) **/pECCG117-metX WT** | 3.51 |

As a result, it was confirmed that O-acetyl-L-homoserine was accumulated with a concentration of 2.83 g/L when the ATCC13032 ΔNCgI2335::PCJ7-yjeH(eco,F351L) ΔmetB ΔmetY lysC(L377K) ΔNCgI0616/pECCG117-metX WT was cultured, and when CM04-8002 was cultured, it was confirmed that the production of O-acetylhomoserine was increased by 130% with a concentration of 3.68 g/L.

Therefore, an increase in the production of O-acetyl-L-homoserine was confirmed when the activity of pyruvate dehydrogenase complex subunit E1 was enhanced in the strain with increased ability to produce O-acetylhomoserine.

Through this, it was confirmed that the production of O-acetylhomoserine was increased when the activity of pyruvate dehydrogenase complex subunit E1 was enhanced in the strain with increased O-acetylhomoserine production.

The above results show that the enhancement of the activity of pyruvate dehydrogenase complex subunit E1 in the O-acetyl-L-homoserine and homoserine-producing strains of the genus *Corynebacterium* is effective in the production of O-acetyl-L-homoserine and derivatives thereof.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the present disclosure is defined by the appended claims rather than by the description preceding them, and therefore all changes and modifications that fall within metes and bounds of the claims, or equivalents of such metes and bounds are therefore intended to be embraced by the claims.

## Claims

1. A method for producing O-acetylhomoserine or a derivative thereof, comprising: culturing a microorganism, in which the activity of pyruvate dehydrogenase complex subunit E1 is increased compared to the endogenous activity thereof, in a medium.

2. The method of claim 1, wherein the microorganism is a microorganism of the genus *Corynebacterium.*

3. The method of claim 2, wherein the microorganism of the genus *Corynebacterium* is *Corynebacterium glutamicum.*

4. The method of claim 1, wherein the pyruvate dehydrogenase complex subunit E1 is derived from *Corynebacterium glutamicum.*

5. The method of claim 1, wherein the pyruvate dehydrogenase complex subunit E1 comprises an amino acid sequence of SEQ ID NO: 1.

6. The method of claim 5, wherein the pyruvate dehydrogenase complex subunit E1 is encoded by a polynucleotide of SEQ ID NO: 2 or SEQ ID NO: 3.

7. The method of claim 1, wherein the O-acetylhomoserine or a derivative thereof is any one or more selected from O-acetylhomoserine, L-methionine, and L-homoserine.

8. The method of claim 1, further comprising a step of recovering O-acetylhomoserine or a derivative thereof from the cultured microorganism, a culture of the microorganism, a fermented product of the microorganism, or the culture medium.

9. A microorganism for producing O-acetylhomoserine or a derivative thereof, in which the activity of pyruvate dehydrogenase complex subunit E1 is increased compared to the endogenous activity thereof.

10. A composition for producing O-acetylhomoserine or a derivative thereof, comprising: a microorganism, in which the activity of pyruvate dehydrogenase complex subunit E1 is increased compared to the endogenous activity thereof; a culture of the microorganism; a fermented product of the microorganism; or a combination of two or more thereof.

11. Use of a microorganism, in which the activity of pyruvate dehydrogenase complex subunit E1 is increased compared to the endogenous activity thereof, for the production of O-acetylhomoserine or a derivative thereof.
